Europäisches Patentamt

**European Patent Office**

**Office européen des brevets**

(19)

(11) Publication number : **0 002 297**

**B1**

(12) **EUROPEAN PATENT SPECIFICATION**

(45) Date of publication of patent specification :
11.02.81

(21) Application number : 78200316.4

(22) Date of filing : 25.11.78

(51) Int. Cl.³ : **C 07 C102/06**, C 07 C103/28,
C 07 C101/18, C 07 B 20/00

(54) Process for the preparation of DL-phenyl-glycine amide.

(30) Priority : 30.11.77 NL 7713189

(43) Date of publication of application :
13.06.79 (Bulletin 79/12)

(45) Publication of the grant of the patent :
11.02.81 Bulletin 81/06

(84) Designated contracting states :
BE CH DE FR GB SE

(56) References cited :
DE - A - 2 319 493
DE - A - 2 345 302
FR - A - 2 334 658
JOURNAL OF THE CHEMICAL SOCIETY,
Perkin Transaction I, n° 4 (1976)
J.C. CLARK et al. « Resolution of Esters of Phenyl-
glycine with (+)-Tartaric Acid », pages 471-474.

CHEMICAL ABSTRACTS,
Vol. 80, n° 5, February 4, 1974, ref. 27471t,
Columbus, Ohio, U.S.A.
T. WATANABE et al. « Racemizing phenylglycine
benzenesulfonate salt », page 447.

(73) Proprietor : STAMICARBON B.V.
Postbus 10
NL-6160 MC Geleen (NL)

(72) Inventor : Boesten, Wilhelmus Hubertus Joseph
Brountslaan 9
NL-6132 BJ Sittard (NL)

(74) Representative : Pinckaers, August René et al
OCTROOIBUREAU DSM Postbus 9
NL-6160 MA Geleen (NL)

Imprimerie Jouve, 17, rue du Louvre, 75001 Paris, France

Process for the preparation of DL-phenyl-glycine amide

The invention relates to a process for the preparation of DL-phenylglycine amide, which is a suitable starting compound for the preparation of D-phenylglycine amide. The latter compound can be converted into D-phenylglycine, which is used especially as a starting material in the synthesis of penicillin compounds. According to a known process (see Netherlands Patent Applications 7 513 551 and 7 700 092), D-phenylglycine amide can be prepared from DL-phenylglycine amide by means of an enzyme preparation. L-phenylglycine is then obtained as a by-product in an amount that is equal in principle to the amount of D-phenylglycine amide. However no uses are known for this by-product wherely the total amount of by-product and the D-phenylglycine amide can be utilised in an economically justified way. A suitable manner has now been found for converting L-phenylglycine into DL-phenylglycine amide.

The process according to the invention for the preparation of DL-phenylglycine amide is characterized in that L-phenylglycine is converted into an ester with an alcohol, the ester is racemized in the presence of a concentrated strong acid, e.g. sulphuric acid, oleum, benzene-sulphonic acids, and p-toluene sulphonic acid, and the resulting DL-ester is converted into DL-phenylglycine amide.

In the process according to the invention, an ester of L-phenylglycine can be prepared by various known methods. A particularly suitable method is the esterification of L-phenylglycine with an alcohol in the presence of a concentrated strong acid, e.g. sulphuric acid, oleum, benzene-sulphonic acid and p-toluene-sulphonic acid, which concentrated strong acid is also used in the racemization of the resulting ester according to the invention. The alcohol used by preference in the esterification is an aliphatic monovalent alcohol with 1-4 carbon atoms. As is usual in esterification, in practice excess alcohol should be present e.g. 5-10 times as much alcohol as is required stoichiometrically. The amount of concentrated acid present during the esterification may be varied, but to obtain good results preferably at least 2.5 equivalents of acid (i.e. 1.25 moles of a dibasic or 2.5 moles of a monobasic acid) are used per mole of phenyl glycine.

To avoid hydrolysis of the ester during racemization, the water content of the concentrated acid should be as low as possible, and preferably should not exceed the water content of 96 % by-weight sulphuric acid. The esterification can readily be carried out at temperatures that are normally used for such a reaction, e.g. a temperature of 50-90 °C.

The racemization of the resulting ester in the presence of a concentrated strong acid can be carried out by heating the ester and the acid, e.g. any of the abovementioned acids, at a temperature between 120 and 200 °C, preferably 140-175 °C, for e.g. 3-10 hours. The pressure used may be atmospheric. Both the esterification and the racemization is preferably carried out in the presence of concentrated sulphuric acid. In order to form the DL-phenylglycine amide from the DL-ester obtained in the racemization, the DL-ester may be separated as such from the reaction mixture obtained in the racemization and be converted into the DL-amide by means of ammonia in known manner. The separation from acid reaction mixture of the DL-ester as such is however not necessary. The entire reaction mixture if desired may be added to aqueous ammonia (e.g. of concentration 10-35 % by-weight of $NH_3$) and the amide be recovered from the resulting reaction mixture, which contains DL-phenylglycine amide and the ammonium salt of the acid used in the racemization, e.g. ammonium sulphate, or the resulting reaction mixture containing amide may be used as such in the enzymatic conversion of DL-phenylglycine amide into D-phenylglycine amide and L-phenylglycine.

The temperature in this amide formation may be any temperature that is usual for the formation of an amide from an ester with ammonia, e.g. a temperature of 20-50 °C. The invention will be further elucidated in the following examples.

Example I

21 millilitres (0.375 mole) of 96 % by-weight sulphuric acid are added with stirring to a solution of 45.3 grams (0.3 mole) of L-phenylglycine in 120 grams of methanol. The mixture is then boiled at atmospheric pressure for 2 hours with reflux cooling. From the resulting reaction mixture, 106.8 grams of methanol-water mixture are distilled at atmospheric pressure, by wich a bottom temperature of 160 °C is reached. The resulting reaction mixture containing ester is subsequently heated at 160 °C and atmospheric pressure for 5 hours with stirring in order to be racemized. 97.2 grams of racemized reaction mixture are obtained. Amino-acid analysis of a sample of the racemized reaction mixture shows that 6.1 % by weight of phenylglycine is contained in this mixture.

A second sample of said reaction mixture is fully hydrolysed by boiling it with reflux cooling for 24 hours with 100 grams of 6 N hydrochloric acid per 0.5 millilitre of sample. Amino-acid analysis of the hydrolysed sample shows that it contains 46.1 % by-weight of phenylglycine.

It can be calculated from the results of the two amino-acid analyses that 86.7 % of the original amount of phenylglycine has been converted into ester.

To determinine the specific rotation of the racemized phenylglycine ester obtained, a 8.5-gram sample of the racemized reaction mixture is hydrolysed (by boiling it with reflux cooling for 6 hours with 100 grams of 6 N sulphuric acid and, after neutralization of the hydrolysed product, separating the phenylglycine) and the specific rotation of the resulting phenylglycine is determined. It amounts to :

$$[\alpha]_D^{20} = + 2.1 \ (c = 1.0 \ ; \ 1.0 \ N \ HCl)$$

Comparison of the measured specific rotation with the corresponding specific rotation of pure L-phenylglycine (+ 157.5°) reveals that 98.6 % of the phenylglycine ester have been racemized.

30 grams of methanol are added to 87.5 grams of the resulting reaction mixture containing DL-phenylglycine ester in order to lower the viscosity of the reaction mixture, after which the resulting solution is slowly added with stirring at 40 °C to 225 millilitres of ammonia water (25 % by weight of NH$_3$) and the mixture obtained is stirred at 40 °C for 4 hours. Next, the ammonia and methanol present are removed by distillation. The remaining reaction mixture (198 grams) is dissolved in water to form 749 grams of solution.

Amino-acid analysis of a sample shows that this solution contains 4.0 % by weight of DL-phenylglycine amide and 1.4 % by weight of DL-phenylglycine. Hence, 85.4 % of the total amount of DL-phenylglycine ester has been converted into the amide.

Example II

42 millilitres (0.75 mole) of 96 % by-weight sulphuric acid is added with stirring to a solution of 90.6 grams (0.6 mole) of L-phenylglycine in 160 grams of methanol). The mixture is then boiled with reflux cooling at atmospheric pressure for 3 hours. From the resulting reaction mixture, 140 grams of methanol-water mixture are distilled at atmospheric pressure, by which a bottom temperature of 140 °C is reached. The resulting reaction mixture containing ester is subsequently heated at 140 °C and atmospheric pressure in order to be racemized. After the resulting racemized reaction mixture (172.3 grams) has cooled, 40 grams of methanol are added.

Amino-acid analysis of a sample of the resulting racemized reaction mixture containing methanol shows 6.4 % by weight of phenylglycine are contained in the reaction mixture obtained in the racemization.

A second sample of said reaction mixture is fully hydrolysed by boiling it with reflux cooling for 24 hours with 100 grams of 6 N hydrochlorid acid per 0.5 millilitre of sample. Amino-acid analysis of the hydrolysed sample shows that it contains 42 % by weight of phenylglycine. It can be calculated from the results of the two amino-acid analyses that 84.8 % of the original amount of phenylglycine has been converted into ester.

To determine the specific rotation of the racemized phenylglycine ester obtained, a 10-gram sample of reaction mixture containing methanol is hydrolysed in the same way as in Example I, after the methanol has been distilled off, and the specific rotation of the resulting phenylglycine is determined. It amounts to :

$$[\alpha]_D^{20} = + 3.8 \ (c = 1.0 \ ; \ 1.0 \ N \ HCl)$$

Comparison of the measured specific rotation with the corresponding specific rotation of pure L-phenylglycine (+ 157.5°) reveals that 97.6 % of the phenylglycine ester have been racemized.

**Claims**

1. Process for the preparation of DL-phenylglycine amide, characterized in that L-phenylglycine is converted into an ester with an alcohol, the ester is racemized in the presence of a concentrated strong acid, and the resulting DL-ester is converted into DL-phenylglycine amide.

2. Process according to claim 1, characterized in that the formation of the ester is effected in the presence of a concentrated strong acid, which acid is also used in the racemization of the ester.

3. Process according to claim 2, characterized in that the concentrated strong acid used is concentrated sulphuric acid.

4. Process according to any one of the claims 1-3, characterized in that the alcohol used is an aliphatic monovalent alcohol with 1-4 carbon atoms.

5. Process according to any one of the claims 1-4, characterized in that the ester is racemized at a temperature of 140-175 °C.

**Ansprüche**

1. Verfahren zur Herstellung von DL-Phenylglycinamid, dadurch gekennzeichnet, dass man L-Phenylglycin mit einem Alkohol in ein Ester umwandelt, das Ester in Anwesenheit von konzentrierter Starksäure razemisiert und das anfallende DL-Ester in DL-Phenylglycinamid umwandelt.

2. Verfahren nach Anspruch 1, dadurch gekennzeichnet, dass man die Bildung des Esters in Anwesenheit einer konzentrierten Starksäure durchführt und diese Säure auch beim Razemisieren des Esters verwendet.

3. Verfahren nach Anspruch 2, dadurch gekennzeichnet, dass man als konzentrierte Starksäure Schwefelsäure gebraucht.

4. Verfahren nach einem der Ansprüche 1-3, dadurch gekennzeichnet, dass man als Alkohol einen aliphatischen einwertigen Alkohol mit 1-4 Kohlenstoffatomen einsetzt.

5. Verfahren nach einem der Ansprüche 1-4, dadurch gekennzeichnet, dass man das Ester bei einer Temperatur von 140-175 °C razemisiert.

**Revendications**

1. Procédé de préparation de DL-phénylglycinamide, caractérisé en ce que la L-phénylglycine est convertie en un ester à l'aide d'un alcool, que l'ester est racémisé en présence d'acide concentré et que le DL-ester obtenu est converti en DL-phénylglycinamide.

2. Procédé selon la revendication 1, caractérisé en ce que la préparation de l'ester est effectuée en présence d'un acide fort concentré et que cet acide-ci est également utilisé pour la racémisation de l'ester.

3. Procédé selon la revendication 2, caractérisé en ce qu'on utilise de l'acide sulfurique comme acide fort concentré.

4. Procédé selon l'une des revendications 1 à 3, caractérisé en ce qu'on utilise comme alcool un alcool monovalent aliphatique avec 1-4 atomes de carbone.

5. Procédé selon l'une des revendications 1 à 4, caractérisé en ce qu'on racémise l'ester à une température de 140-175 °C.